# EUROPEAN PATENT APPLICATION

(11) **EP 2 781 503 A1**
(43) Date of publication of application: **24.09.2014**
(21) Application number: 13159729.6
(22) Date of filing: 18.03.2013
(51) Int. Cl.: C07C 68/00

(54) **Oxidative carbonylation of monohydroxy aryl compounds by methyl formate**

(71) Applicant: Bayer MaterialScience AG, 51373 Leverkusen (DE)
(72) Inventor: Yalfani, Mohammad, 52062 Aachen (DE); Lolli, Giulio, 51069 Köln (DE); Wolf, Aurel, 42489 Wülfrath (DE); Mleczko, Leslaw, 41542 Dormagen (DE)
(74) Representative: BIP Patents

(57) **Abstract**

The field of the present invention relates to a process for continuously preparing alkylaryl carbonates and diarylcarbonates from methyl formate and at least one monohydroxy aryl compound in the presence of catalysts, and to the use thereof for preparation of polycarbonates.

## Description

The field of the present invention relates to a process for continuously preparing alkylaryl carbonates and diarylcarbonates from methyl formate and at least one monohydroxy aryl compound in the presence of catalysts, and to the use thereof for preparation of polycarbonates.

Methyl formate was used as a green and efficient carbonylating agent, instead of toxic CO gas, in the oxidative carbonylation of monohydroxy aryl compound to methyl aryl carbonate. Methyl formate showed also better performance and the ability to produce other useful carbonylated products, e.g., dimethyl carbonate and dimethyl oxalate.

The oxidative carbonylation of phenol to diphenyl carbonate and other carbonates has been studied extensively during the recent decades as described for example in; K. J. L. Linsen, J. Libens and P. A. Jacobs, Chem. Commun. 2002, 2728; L. Ronchin, A. Vavasori, E. Amadio, G. Cavinato and L. Toniolo, J. Mol. Catal. A: Chem. 2009, 298, 23; X. Yang, J. Han, Z. Du, H. Yuan, F. Jin and Y. Wu, Catal. Commun. 2010, 11, 643; Y. Zhao, Q. Jia, Y. Liang, H. Guo, F. Li and X. Liu, Adv. Mater. Res. 2011, 284-286, 707; T. Murayama, T. Hayashi, R. Kanega and I. Yamanaka, J. Phys. Chem. C, 2012, 116, 10607. The method can be one of the promising alternatives to replace the current industrial process of diphenyl carbonate production (DPC) as described in J. Gong, X. Ma and S. Wang, Appl. Catal. A: Gen., 2007, 316, 1. The later product is a key component for the manufacturing of polycarbonates. However, this process needs to be improved due to hazard problems related to the handling of toxic CO gas according to T. Morimoto and K. Kakiuchi, Angew. Chem., Int. Ed. 2004, 43, 5580. Thus, a process involving a safer and more sustainable carbonylating agent is preferred for industrial applications.

Methyl formate has been widely utilized as C₁ building block in organic synthesis as for example in J. S. Lee, J. C. Kim and Y. G. Kim, Appl. Catal. 1990, 57, 1. It can be activated using an appropriate catalyst to take part in carbonylation reactions. Activation of methyl formate can be effected through carbonyl-hydrogen bond dissociation or a C-O-C bond cleavage. Transition metals like Ru and Ir are able to activate methyl formate *via* carbonyl-hydrogen bond dissociation, which leads to direct methoxylcarbonylation (see G. Jenner, E. M. Nahmed and H. Leismann, J. Organometal. Chem. 1990, 387, 315; C. Legrand, Y. Castanet, A. Mortreux and F. Petit, J. Chem. Soc. Chem. Commun. 1994, 1173). This process usually requires temperatures higher than 180 °C. Activation of methyl formate by strong bases (e.g., NaOCH₃) results in partial decarbonylation to CO and CH₃OH at low temperatures (40-80 °C) as shown in J. -F. Carpentier, Y. Castanet, J. Brocard, A. Montreux and F. Petit, Tetrahedron Lett. 1991, 32, 4705. Given a very week acidity of methyl formate (see S. Ingemann and N. M. M. Nibbering, J. Org. Chem. 1985, 50, 682), the hydrogen bound to the carbonyl group behaves as a proton source against strongly basic methoxide. Pennequin *et al.* used methyl formate in the oxidative carbonylation of alkenes employing PdCl₂(PPh₃)₂ catalyst in P. Pennequin, M. Fontaine, Y. Castanet, A. Mortreux and F. Petit, Appl. Catal. A: Gen. 1996, 135, 329. They showed that the key steps in this reaction are (1) decarbonylation of methyl formate by NaOCH₃, (2) formation of a methoxycarbonyl-Pd complex, (3) insertion of alkene and (4) β-hydride elimination giving the olefinic ester. In addition, an initial CO pressure (*ca.* 15 bar) is essential for this reaction.

There was therefore a need for a method for producing alkylaryl carbonates and diarylcarbonates that provides high security standards and employs "green" reactants.

This problem was solved by using Methyl formate (methyl formate) as a green and efficient carbonylating agent in the oxidative carbonylation of monohydroxy aryl compound, preferably phenol to methyl aryl carbonate, preferably methyl phenyl carbonate, in the presence of a catalyst. In a preferred embodiment, a co-catalyst is used. In a further preferred embodiment, activation of methyl formate is catalysed by alkali metal alkoxides MOCH₃, wherein M is Na or K.

The preferred catalysts in the present invention are redox catalysts, preferably on Palladium basis. Particularly preferred catalysts are PdBr₂, Pd(OAc)₂, Pd(salen) (salen: *N,N*'-bis(salicylidene)-ethylenediamine), Pd(10%)/C, Pd(5%)/C, Pd(OAc)₂, PdBr₂, Pd(OAc)₂, PdCl₂(CH₃CN)₂.

Co-catalysts according to the preferred embodiment are on basis of manganese, cerium or copper, particularly preferably Mn(acac)₃, Ce(OAc)₂, CuCl₂, Cu(OAc)₂.

Monohydroxyl compounds which are suitable in the context of the invention are preferably those of the general formula (VIII) 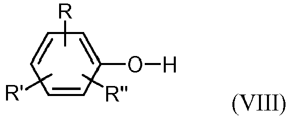 in which R, R' and R" may each independently be as defined for the general formula (VII).

Such aromatic monophenols are, for example: phenol, o-, m- or p-cresol, also as a mixture of the cresols, dimethylphenol, also as a mixture, where the position of the methyl groups on the phenol ring may be as desired, e.g. 2,4-, 2,6-, or 3,4-dimethylphenol, o-, m- or p-chlorophenol, o-, m- or p-ethylphenol, o-, m- or p-n-propylphenol, 4-isopropylphenol, 4-n-butylphenol, 4-isobutylphenol, 4-tert-butylphenol, 4-n-pentylphenol, 4-n-hexylphenol, 4-isooctylphenol, 4-n-nonylphenol, o-, m- or p-methoxyphenol, 4-cyclohexylphenol, 4-(1-methyl-1-phenylethyl)phenol, biphenyl-4-ol, 1-naphthol, 2-1-naphthol, 4-(1-naphthyl) phenol, 4-(2-naphthyl)phenol, 4-phenoxyphenol, 3-pentadecylphenol, 4-tritylphenol, methylsalicylic acid, ethylsalicylic acid, n-propylsalicylic acid, isopropylsalicylic acid, n-butylsalicylic acid, isobutylsalicylic acid, tert-butylsalicylic acid, phenylsalicylic acid and benzylsalicylic acid.

Preferred monophenols are phenol, 4-tert-butylphenol, biphenyl-4-ol and 4-(1-methyl-1-phenylethyl)phenol.

Particular preference is given to phenol.

Methyl aryl carbonates obtained as intermediates in the context of the invention are preferably those of the general formula (IX) 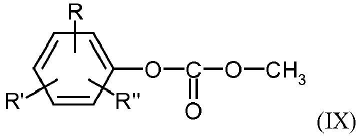 in which R, R' and R" may each be as defined for the general formula (VII).

Preferred alkyl aryl carbonates are methyl phenyl carbonate, methyl p-chlorophenyl carbonate, methyl o-cresyl carbonate, methyl p-cresyl carbonate. Particularly preferred alkyl aryl carbonate is methyl phenyl carbonate.

Diaryl carbonates prepared in the context of the invention are preferably those of the general formula (VII) 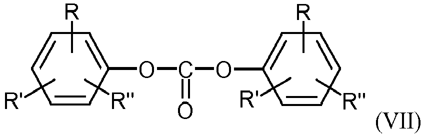 where R, R' and R" are each independently H, linear or branched, optionally substituted C₁-C₃₄-alkyl, preferably C₁-C₆-alkyl, more preferably C₁-C₄-alkyl, C₁-C3₄-alkoxy, preferably C₁-C₆-alkoxy, more preferably C₁-C₄-alkoxy, C₅-C₃₄-cycloalkyl, C₇-C₃₄-alkylaryl, C₆-C₃₄-aryl or a halogen radical, preferably a chlorine radical, and R, R' and R" on both sides of the formula (VII) may be the same or different. R may also be -COO-R'" where R'" may be H, optionally branched C₁-C₃₄-alkyl, preferably C₁-C₆-alkyl, more preferably C₁-C₄-alkyl, C₁-C₃₄-alkoxy, preferably C₁-C₆-alkoxy, more preferably C₁-C₄-alkoxy, C₅-C₃₄-cycloalkyl, C₇-C₃₄-alkylaryl or C₆-C₃₄-aryl. Preferably, R, R' and R" on both sides of the formula (VII) are the same. Most preferably, R, R' and R" are each H.

Diaryl carbonates of the general formula (VII) are, for example: diphenyl carbonate, methylphenyl phenyl carbonates and di(methylphenyl) carbonates, also as a mixture, where the position of the methyl group on the phenyl rings may be as desired, and also dimethylphenyl phenyl carbonates and di(dimethylphenyl) carbonates, also as a mixture, where the position of the methyl groups on the phenyl rings may be as desired, chlorophenyl phenyl carbonates and di(chlorophenyl) carbonates, where the position of the methyl group on the phenyl rings may be as desired, 4-ethylphenyl phenyl carbonate, di(4-ethylphenyl) carbonate, 4-n-propylphenyl phenyl carbonate, di(4-n-propylphenyl) carbonate, 4-isopropylphenyl phenyl carbonate, di(4-isopropylphenyl) carbonate, 4-n-butylphenyl phenyl carbonate, di(4-n-butylphenyl) carbonate, 4-isobutylphenyl phenyl carbonate, di(4-isobutylphenyl) carbonate, 4-tert-butylphenyl phenyl carbonate, di(4-tert-butylphenyl) carbonate, 4-n-pentylphenyl phenyl carbonate, di(4-n-pentylphenyl) carbonate, 4-n-hexylphenyl phenyl carbonate, di(4-n-hexylphenyl) carbonate, 4-isooctylphenyl phenyl carbonate, di(4-isooctylphenyl) carbonate, 4-n-nonylphenyl phenyl carbonate, di(4-n-nonylphenyl) carbonate, 4-cyclohexylphenyl phenyl carbonate, di(4-cyclohexylphenyl) carbonate, 4-(1-methyl-1-phenylethyl)phenyl phenyl carbonate, di[4-(1-methyl-1-phenylethyl)phenyl] carbonate, biphenyl-4-yl phenyl carbonate, di(biphenyl-4-yl) carbonate, 1-naphthyl phenyl carbonate, 2-naphthyl phenyl carbonate, di(1-naphthyl) carbonate, di(2-naphthyl) carbonate, 4-(1-naphthyl)phenyl phenyl carbonate, 4-(2-naphthyl)phenyl phenyl carbonate, di[4-(1-naphthyl)phenyl] carbonate, di[4-(2-naphthyl)phenyl] carbonate, 4-phenoxyphenyl phenyl carbonate, di(4-phenoxyphenyl) carbonate, 3-pentadecylphenyl phenyl carbonate, di(3-pentadecylphenyl) carbonate, 4-tritylphenyl phenyl carbonate, di(4-tritylphenyl) carbonate, (methyl salicylate) phenyl carbonate, di(methyl salicylate) carbonate, (ethyl salicylate) phenyl carbonate, di(ethyl salicylate) carbonate, (n-propyl salicylate) phenyl carbonate, di(n-propyl salicylate) carbonate, (isopropyl salicylate) phenyl carbonate, di(isopropyl salicylate) carbonate, (n-butyl salicylate) phenyl carbonate, di(n-butyl salicylate) carbonate, (isobutyl salicylate) phenyl carbonate, di(isobutyl salicylate) carbonate, (tert-butyl salicylate) phenyl carbonate, di(tert-butyl salicylate) carbonate, di(phenyl salicylate) carbonate and di(benzyl salicylate) carbonate.

Preferred diaryl carbonates are: diphenyl carbonate, 4-tert-butylphenyl phenyl carbonate, di(4-tert-butylphenyl) carbonate, biphenyl-4-yl phenyl carbonate, di(biphenyl-4-yl) carbonate, 4-(1-methyl-1-phenylethyl)phenyl phenyl carbonate and di[4-(1-methyl-1-phenylethyl)phenyl] carbonate.

Particular preference is given to diphenyl carbonate.

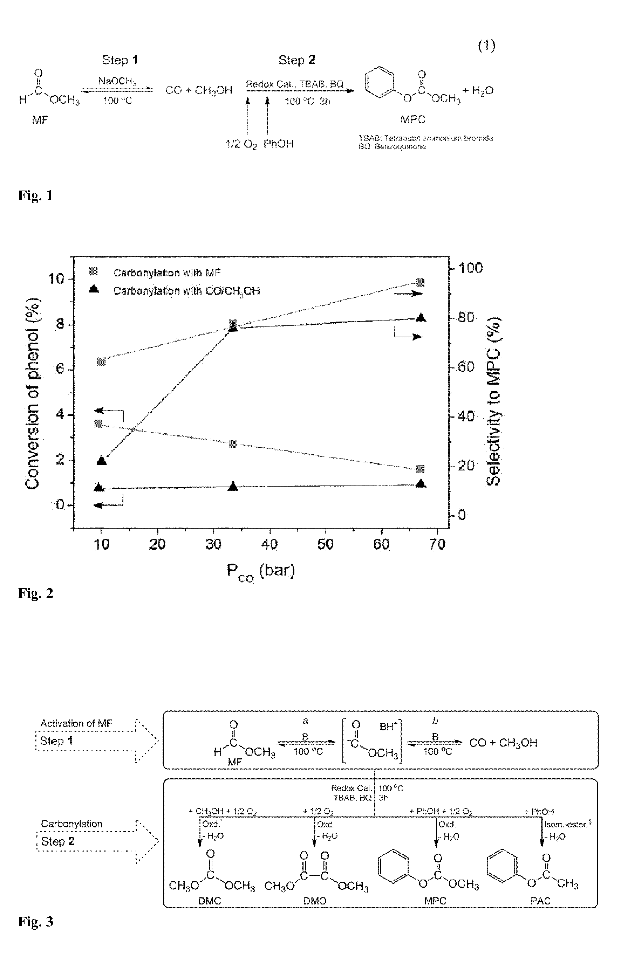

Methyl formate was utilized as a carbonylating agent for phenol to obtain methyl phenyl carbonate (MPC) as the target product. MPC, can be subsequently converted to DPC by an esterification with phenol. There is no report on the application of methyl formate in the oxidative carbonylation of monohydroxy aryl compound phenol in literature. As phenol has much higher acidic character than methyl formate, it reacts faster with methoxide. Therefore, in order to avoid the interference of phenol, the activation of methyl formate was separated from the carbonylation of phenol as summarized in Fig. 1. The reaction was performed in two consecutive steps in the range of 80 to 120 °C, preferably at 100 °C. Activation of methyl formate was catalysed here by alkali metal alkoxides MOCH₃ (M = Na or K) which resulted in increasing the internal pressure (Pᵢₙ) of the reactor. This Pᵢₙ was generated by the partial decarbonylation of methyl formate and, thus, can be attributed to the formation of CO.

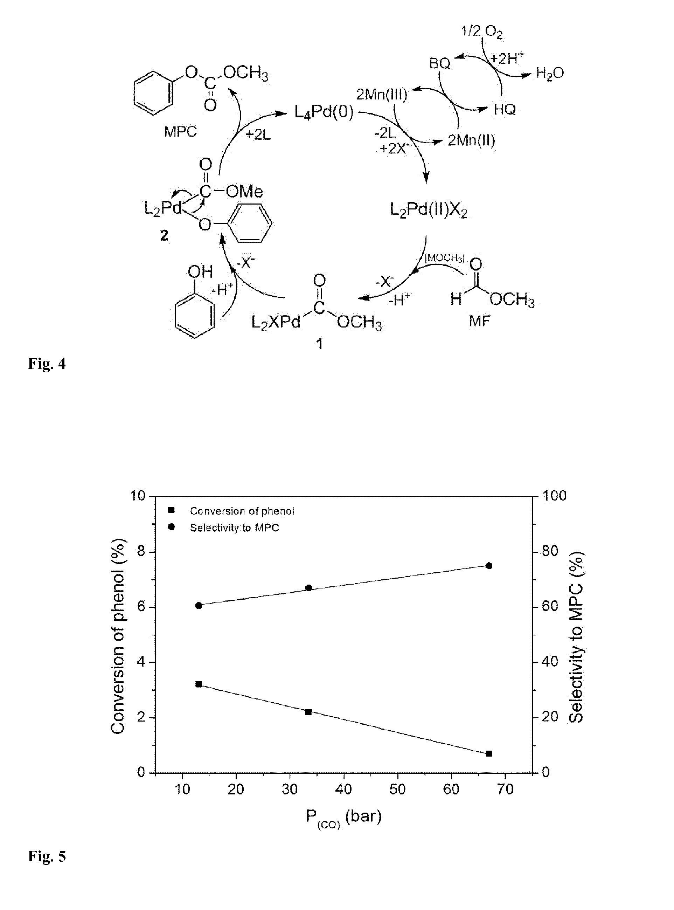

Concerning the MPC production and its side products, a mechanism for the process is proposed as shown in Fig. 4. methyl formate is activated by MOCH₃ *via* carbonyl-hydrogen bond dissociation, which in the presence of Pd(II) leads to the formation of methoxycarbonyl-palladium intermediate **1.** This complex is very stable and depending on the type of ligand can even be formed at room temperature. Activation of methyl formate by MOCH₃ can be represented by the equilibrium equation in Fig. 3 - step 1. The intermediate in this equilibrium is a methoxycarbonyl ion resulting from the dissociation of the carbonyl-hydrogen bond. At low amounts of MOCH₃ (e.g., NaOCH₃ - 4.68 mmol corresponding to an initial CO pressure of *ca.* 10 bar), a longer lifetime for methoxycarbonyl is expected.

This means that the kinetics of the activation of methyl formate dominates the decarbonylation towards CO + CH₃OH. Therefore, at low initial CO pressures, methoxycarbonyl is the main agent for the formation of **1.** At higher amounts of NaOCH₃, the equilibrium in step 1 shifts to the right, i.e., CO + CH₃OH. Thus, **1** can also be formed by a consecutive methoxylation and carbonylation. However, since MPC production by the comparative reaction using CO + CH₃OH was less efficient, it can be concluded that the formation of **1** is favoured through activation of methyl formate. In the next step, **1** reacts with a phenolate to form the intermediate **2.** Finally, methoxycarbonyl donates electrons to Pd(II) and reacts with phenolate, forming MPC. Simultaneously, Pd(II) is reduced to metallic Pd(0), which is then re-oxidized by Mn(III) to complete the cycle. The general aspects of this mechanism are similar to the mechanism proposed for the oxidative carbonylation of phenol by CO and O₂. It also simulates a Wacker-type catalytic reaction.

Methyl formate can undergo isomerization to acetic acid, which in the presence of phenol leads to PAC through esterification. At low CO pressures, isomerization of methyl formate to acetic acid is more plausible, leading to formation of higher amounts of PAC.

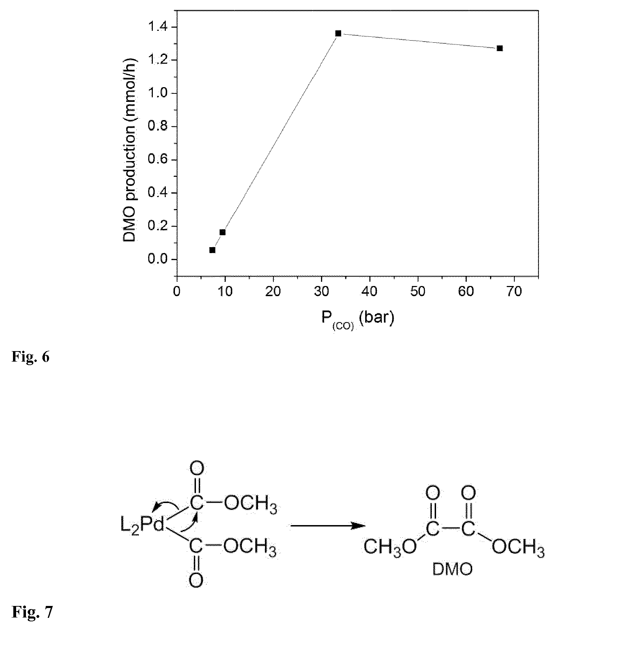

DMO is produced by reacting a second methoxycarbonyl group to Pd, followed by coupling of two methoxycarbonyl groups. This can also be the result of an oxidation reaction. As the quantitative results show (Fig. 6), at CO pressures lower than 33 bar, the formation of dimethyl oxalate is drastically reduced. Probably, for the reaction of two methoxycarbonyl groups with Pd generating the Pd(COOCH₃)₂ intermediate (see Fig. 7), a relatively high CO pressure is required. DMC is also formed as a result of methoxide reacting with **1.** Then methoxycarbonyl and methoxide are combined through an oxidative carbonylation reaction.

In summary, the formation of MPC by oxidative carbonylation of phenol with methyl formate was achieved. The process does not need an additional source of CO gas. Using excessive amount of NaOCH₃, decarbonylation of methyl formate dominates and the initial CO pressure reaches equilibrium values of 67-69 bar. At this high initial CO pressure, although, the conversion of phenol is low (ca. 1.5-2%), the selectivity to MPC is high (*ca.* 95%). By decreasing the amount of NaOCH₃ and consequently adjusting the kinetic regime of methyl formate activation, the conversion of phenol increases to as high as 6%, while the selectivity to MPC was maintained at *ca.* 60%. Most importantly, the use of methyl formate showed higher efficiency in the carbonylation of phenol than the conventional CO + CH₃OH system. Two further oxidative carbonylation reactions occur in parallel, which lead to the formation of DMO and DMC, two other useful products for the DPC production. Thus, methyl formate is a safe and promising substitute for gaseous CO in oxidative carbonylation processes.
**Fig. 1** separation of the activation of methyl formate from the carbonylation of phenol
**Fig. 2** Phenol conversion and MPC selectivity obtained by carbonylation of phenol by either methyl formate or CO + CH₃OH whereby the initial CO pressure was varied.
**Fig. 3** Reaction pathways during the oxidative carbonylation of phenol by methyl formate
**Fig. 4** Mechanism of carbonylation of phenol to MPC by methyl formate using Pd(II) catalysts (L: neutral ligand, X: ionic ligand like Br-, OAc-) and Mn(acac)₃ as co-catalyst
**Fig. 5** Oxidative carbonylation of phenol with methyl formate using KOCH₃ as catalyst for the activation of methyl formate
**Fig. 6** DMO production vs. the initial CO pressure during the oxidative carbonylation of phenol with methyl formate
**Fig. 7** The structure of the Pd(COOCH₃)₂ complex and the formation of DMO during the oxidative carbonylation of phenol with methyl formate

### Examples

### Measurement methods

The method used for GC analysis was as follows:
Instrument: Thermo SCIENTIFIC TRACE GC Ultra, column: OV1-IVA (50 m × 0.25 mm × 0.5 µm); Conditions: 50-250 °C, 5 min isothermal, 8 °C/min, 30 min isothermal; FID 250 °C; t_{PhOH}: 14.26 min, t_{MPC}: 17.71, t_{DMO}: 10.08 min.

The method used for GC-MS analysis was as follows:
Instrument: VARIAN CP3800 gas chromatograph - VARIAN 1200L Quadrupole MS/MS; column: SE34 (30m × 0.32 mm); Conditions: 50-250 °C, 5 min isothermal, 8 °C/min, 30 min isothermal; FID 250 °C.

### General conditions for the production of Methyl Phenyl Carbonate

The reaction was performed in two consecutive steps at 100 °C. Activation of methyl formate was catalysed here by alkali metal alkoxides MOCH₃ (M = Na or K) which resulted in increasing the internal pressure (Pᵢₙ) of the reactor. This Pᵢₙ was generated by the partial decarbonylation of methyl formate and, thus, can be attributed to the formation of CO.

Various well-known Pd(II) catalysts were screened together with Mn(acac)₃ as co-catalyst in order to study their performance in the carbonylation of phenol by methyl formate.

Pᵢₙ at the beginning of the carbonylation of phenol reached 67-69 bar. As the results in Table 1 show, a low conversion for phenol along with a high selectivity to MPC was obtained. The other phenolic product is phenyl acetate (PAC), which is the result of isomerization of methyl formate, followed by the esterification with phenol. Among these Pd catalysts, PdBr₂, Pd(OAc)₂ and Pd(salen) showed a selectivity higher than 90% for MPC. In compsrison, other redox couples, such as PdBr₂-CuCl₂, showed better selectivity to MPC (97%). Reactions using homogeneous catalysts, i.e., Pd(OAc)₂-Cu(OAc)₂ and PdCl₂(CH₃CN)₂-Cu(OAc)₂ showed higher conversion of phenol (3.6% and 2.3% respectively), while the selectivity to MPC was lower (57-59%).

### 1. Experimental details for the oxidative carbonylation of phenol with MF

The optimized protocol for the carbonylation of phenol by MF is as following: in the first step, a 160 mL Parr autoclave reactor is charged with MF (45 ml = 734 mmol), NaOCH₃ (4.68-9.36 mmol), Pd(II) catalyst (0.11 mmol), inorganic co-catalyst (0.6875 mmol), tetrabutyl ammonium bromide (TBAB) (1.7 mmol), benzoquinone (BQ) (0.250 mmol) and molecular sieves (MS) 3Å (1 g). In the second step, after the temperature of the reaction had stabilized at 373 K and the internal pressure (Pᵢₙ) became almost constant, the reactor was pressurized by O₂ (7% of Pᵢₙ at 373 K). Immediately phenol (9.09 mmol) dissolved in CH₂Cl₂ (10 mL) was injected into the reactor (3.5 mL/min) using a high pressure HPLC pump. The injection of phenol was taken as time zero. After 3h, the reaction was quenched by ice-water. The reaction products were analysed by GC and GC-MS spectroscopy. TBAB was used as a stabilizer for Pd(II) and BQ was an organic co-catalyst, which facilitated the redox cycle.

**Table 1 Carbonylation of phenol by MF using different redox catalysts ^{a}**

| | | | | |
|---|---|---|---|---|
| No. | Catalyst | Co-catalyst | Conv. (%) *^{b}* | Select. (%)*^{c}* |
| 1 | PdBr₂ | Mn(acac)₃ | 1.6 | 94.5 |
| 2*^{d}* | PdBr₂ | Mn(acac)₃ | 1.4 | 93.6 |
| 3*^{e}* | PdBr₂ | Mn(acac)₃ | 1.1 | 94.4 |
| 4 | Pd(OAc)₂ | Mn(acac)₃ | 1.7 | 95.2 |
| 5 | Pd(salen)*^{f}* | Mn(acac)₃ | 1.7 | 91.3 |
| 6 | Pd(10%)/C | Mn(acac)₃ | 1.1 | 83.7 |
| 7 | Pd(5%)/C | Mn(acac)₃ | 1.7 | 75.4 |
| 8 | Pd(OAc)₂ | Ce(OAc)₂ | 1.8 | 85.0 |
| 9 | PdBr₂ | CuCl₂ | 1.3 | 97.0 |
| 10 | Pd(OAc)₂ | Cu(OAc)₂ | 3.6 | 57.9 |
| 11 | PdCl₂(CH₃CN)₂ | Cu(OAc)₂ | 2.3 | 59.7 |

| | | | | |
|---|---|---|---|---|
| *^{a}* For experimental details, see Table S1, ESI†; *^{b}* conversion of phenol; *^{c}* Selectivity to MPC; *^{d}* solvent: dimethyl formamide; *^{e}* solvent: tetrahydrofuran; *^{f}* salen: *N,N*'-bis(salicylidenyl)-ethylenediamine. | | | | |

As mentioned above, Pᵢₙ at the beginning of the second step, represented the equilibrium CO pressure, generated by the decarbonylation of methyl formate with NaOCH₃ (see step 1 in equation 1). In other words, Pᵢₙ was the initial CO pressure for the carbonylation reaction. The effect of the initial CO pressure on the carbonylation of phenol by methyl formate was studied by adjusting the amount of NaOCH₃ in the range of 4.68-9.36 mmol thereby controlling the rate of the decarbonylation of methyl formate. In consequence, the initial CO pressure was controlled within the range of 10-67 bar. As shown in Fig. 1, by reducing the initial CO pressure from 67 to 10 bar, the conversion of phenol increased while the selectivity to MPC decreased. Reducing the NaOCH₃ amount to lower than 4.68 mmol, resulting in initial CO pressures of less than 10 bar *(ca.* 8 bar), led to even higher conversion of phenol (up to 6%), while the high selectivity to MPC (60%) was maintained. At amounts lower than 4.68 mmol (initial CO pressures less than 7 bar), phenol conversion decreased drastically and the selectivity to MPC dropped to less than 5%. In this case, most of the phenol was converted to PAC.

The decarbonylation of methyl formate was also performed using KOCH₃, which is a stronger base than NaOCH₃. For this reason, lower amounts of KOCH₃ were required to achieve similar initial CO pressures. By varying the amount of KOCH₃ within the range of 2.5-6 mmol, the CO pressure was adjusted in the range of 13-67 bar. As the reactions with NaOCH₃, by reducing the initial CO pressure from 67 to 13, an increase (from less than 1% to more than 3%) in phenol conversion and a decrease (from 75% to 60%) in MPC selectivity were observed (see Fig. 5). The reaction did not proceed using LiOCH₃, most likely, as it is a weaker base than NaOCH₃, and is not able to activate/decarbonylate methyl formate at 100 °C.

### 2. Experimental details for the oxidative carbonylation of phenol with CO+CH₃OH

The efficiency of methyl formate in the carbonylation of phenol was studied by performing comparative tests, whereby CO + CH₃OH was used instead of methyl formate under the same reaction conditions.

Similar to the reactions with MF, a 160 mL Parr autoclave reactor was charged with MeOH (45 ml = 1.11 mol), NaOCH₃ (4.68 mmol), Pd(II) catalyst (0.11 mmol), inorganic co-catalyst (0.6875 mmol), tetrabutyl ammonium bromide (TBAB) (1.7 mmol), benzoquinone (BQ) (0.250 mmol) and molecular sieves (MS 3Å) (1 g). Then after the temperature had stabilized at 373 K, the reactor was pressurized first with CO and then O₂ to the desired pressure (O₂ was 7% of CO pressure). Immediately phenol (9.09 mmol) dissolved in CH₂Cl₂ (10 mL) was injected into the reactor (3.5 mL/min) using a high pressure HPLC pump. The injection of phenol was taken as time zero. After 3h, the reaction was quenched by ice-water. The reaction products were analysed by GC and GC-MS spectroscopy. TBAB was used as a stabilizer for Pd(II) and BQ was an organic co-catalyst, which facilitates the redox cycle.

The results obtained using different CO pressures are in Fig. 1. Phenol conversion under such conditions was independent of the CO pressure. Most importantly, phenol conversion in all cases was lower than when methyl formate was used. The selectivity to MPC decreased by decreasing the CO pressure, in particular, for pressures as low as 10 bar. These results indicate better performance of methyl formate in the carbonylation reaction in comparison to the conventional CO + CH₃OH system. Further, it helped us find a reliable mechanism for the carbonylation of phenol by methyl formate.

Other than MPC and PAC, dimethyloxalate (DMO) and dimethylcarbonate (DMC) were identified as side-products by GC-MS. DMO and DMC are also known as intermediates for DPC. DMO is the result of oxidative self-carbonylation of methyl formate while DMC is the result of oxidative carbonylation of methanol (the product of decarbonylation of methyl formate). No DPC was detected in the final reaction medium. A overview to the whole process involving the pathways to each product is shown in Fig. 3 including three oxidation and one isomerization-esterification reactions.

## Claims

1. Method for producing alkylaryl carbonates, wherein methyl formate and at least one monohydroxy aryl compound are reacted in presence of at least one catalyst.

2. Method for producing alkylaryl carbonates according to claim 1, wherein at least one catalyst is a redox catalysts.

3. Method for producing alkylaryl carbonates according to claim 2, wherein, the redox catalyst is on Palladium basis.

4. Method for producing alkylaryl carbonates according to claim 3, wherein, the redox catalyst is selected from PdBr₂, Pd(OAc)₂, Pd(salen) (salen: *N,N'*-bis(salicylidene)-ethylenediamine), Pd(10%)/C, Pd(5%)/C, Pd(OAc)₂, PdBr₂ and Pd(OAc)₂, PdCl₂(CH₃CN)₂.

5. Method for producing alkylaryl carbonates according to claim 1, wherein further to the catalyst, a co-catalyst is used.

6. Method for producing alkylaryl carbonates according to claim 5, wherein the co-catalyst is on basis of manganese, cerium or copper.

7. Method for producing alkylaryl carbonates according to claim 6, wherein the co-catalyst is Mn(acac)₃, Ce(OAc)₂, CuCl₂ or Cu(OAc)₂.

8. Method for producing alkylaryl carbonates according to one of the preceding claims, wherein the methyl formiate is activated by an alkali metal alkoxides MOCH₃.

9. Method for producing alkylaryl carbonates according to claim 3, wherein M is Na or K

10. Method for producing alkylaryl carbonates according to one of the preceeding claims, wherein the monohydroxyl compound is of the general formula (VIII) 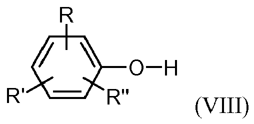 where R, R' and R" are each independently H, linear or branched, optionally substituted C₁-C₃₄-alkyl, preferably C₁-C₆-alkyl, more preferably C₁-C₄-alkyl, C₁-C₃₄-alkoxy, preferably C₁-C₆-alkoxy, more preferably C₁-C₄-alkoxy, C₅-C₃₄-cycloalkyl, C₇-C₃₄-alkylaryl, C₆-C₃₄-aryl or a halogen radical, preferably a chlorine radical, and R, R' and R" on both sides of the formula (VIII) may be the same or different.

11. Method for producing alkylaryl carbonates according to one of the preceeding claims, wherein the monohydroxyl compound is phenol, 4-tert-butylphenol, biphenyl-4-ol and 4-(1-methyl-1-phenylethyl)phenol.

12. Alkylaryl carbonate obtainable by the method according to one of the previous claims.

13. Method for producing diarylcarbonates from the alkylaryl carbonate according to claim 12.

14. Method for producing polycarbonate by using a diarylcarbonate according to claim 13.
